# EUROPÄISCHE PATENTANMELDUNG

(11) **EP 1 709 989 A1**
(43) Veröffentlichungstag der Anmeldung: **11.10.2006**
(21) Anmeldenummer: 05102643.3
(22) Anmeldetag: 04.04.2005
(51) Int. Cl.: A61M 37/00

(54) **Tätowierpistolenspitze**

(71) Anmelder: Christoph Burckhardt AG, 4057 Basel (CH)
(72) Erfinder: Ulmer, Christoph, 4054 Basel (CH); Burckhardt, Theodor, 4144 Arlesheim (CH)
(74) Vertreter: Jönsson, Hans-Peter

(57) **Zusammenfassung**

Eine Vorrichtung (10) zum Entfernen einer Tätowierung weist eine Tätowierpistolenspitze (14) und eine Tätowierpistole (12) auf. Die Tätowierpistolenspitze (12) weist einen Spitzenkörper (28) auf, der einen Nadelkanal (32) zur Führung mindestens einer Tätowiernadel (34) aufweist. Der Spitzenkörper (28) ist mit einem Vorratsbehälter (50) zur Aufnahme einer Flüssigkeit verbunden. Erfindungsgemäß ist der Vorratsbehälter (50) mit einem Hautirritationsmittel (52) gefüllt. Ferner ist der Vorratsbehälter (50) über einen Zuführkanal zum Benetzen der Tätowiernadel (34) mit dem Hautirritationsmittel (52) mit dem Nadelkanal (32) verbunden, wobei der Zuführkanal (54) in den Nadelkanal (32) einmündet. Dadurch werden unnötige Totvolumina vermieden, so dass der Verbrauch des Hautirritationsmittels minimiert ist.

## Beschreibung

Die Erfindung betrifft eine Tätowierpistolenspitze für Tätowierpistolen, die zur Entfernung von Tätowierungen verwendet werden.

Aus EP 1 330 199 B1 ist eine Vorrichtung zum Entfernen einer Tätowierung der menschlichen oder tierischen Haut bekannt. Diese Vorrichtung weist eine Tätowierpistole mit mindestens einer Nadel auf. Zur Entfernung der Tätowierung wird die mindestens eine Nadel zunächst in ein Behältnis getunkt, in dem ein Hautirritationsmittel angeordnet ist. Anschließend durchsticht die mit dem Hautirritationsmittel benetzte Nadel die Haut im Bereich der Tätowierung, wodurch die relativ großen Farbpigmentagglomerate der Tätowierung zerstochen werden. Beim Durchstechen der Haut bleibt ein Teil des Hautirritationsmittels in dem Stichkanal der Nadel zurück, so dass die zerkleinerten Farbpigmentagglomerate durch den natürlichen Heilungsprozess der Haut nach außen abgetragen werden können.

Nachteilig bei einer derartigen Tätowierpistole ist, dass das Eintauchen der Tätowierpistolenspitze zum Benetzen der Nadel in das das Hautirritationsmittel aufweisende Behältnis mühsam und zeitaufwändig ist. Ferner ist es erforderlich, dass die Nadel über den gesamten Bereich, der in die Haut eingestochen wird, mit dem Hautirritationsmittel benetzt ist, damit eine zu schnelle Verheilung des Einstechkanals, die den Austrag der zerkleinerten Farbpigmentagglomerate verhindern würde, vermieden ist. Beim Eintauchen der Nadel in das Hautirritationsmittel besteht jedoch die Gefahr, dass eine ausreichende Benetzung der Nadel nicht erfolgt. Diese Gefahr ist umso größer, je niedriger der Füllstand in dem Behältnis ist. Um die Gefahr einer unzureichenden Benetzung der Nadel zu verringern, ist es erforderlich, das nur noch teilweise mit dem Hautirritationsmittel gefüllte Behältnis nicht mehr zu verwenden. Da bei der Entfernung von Tätowierungen hohe Hygieneanforderungen bestehen, um beispielsweise Infektionen zu vermeiden, kann das Behältnis mit dem noch verbliebenen Hautirritationsmittel nicht wiederverwertet werden. Der Verbrauch an Hautirritationsmittel zur Entfernung einer Tätowierung ist somit sehr hoch.

Aus JP 2003 339 875 A ist eine Tätowierpistole mit einer Tätowierpistolenspitze bekannt. Die Tätowierpistolenspitze weist einen Spitzenkörper auf, der im Wesentlichen rohrförmig mit einem kegelstumpfartigen Abschnitt am zur Haut weisenden Ende der Tätowierpistolenspitze ausgebildet ist. Bevor der rohrförmige Bereich in den kegelstumpfartigen Bereich übergeht, ist in dem rohrförmigen Bereich eine Öffnung vorgesehen, um Tinte aus einem Vorratsbehälter in den Spitzenkörper einzufüllen. Die eingefüllte Tinte sammelt sich im kegelstumpfförmigen Bereich des Spitzenkörpers und kann dort die Nadel benetzen. Anstelle von Tinte kann auch das Bleichmittel H₂O₂ in den Spitzenkörper eingefüllt werden, um Tätowierungen zu entfernen. Um eine Tätowierung vollständig auszubleichen, ist es erforderlich, verhältnismäßig viel Flüssigkeit in Kontakt mit den Farbpigmentagglomeraten der Tätowierung zu bringen, so dass die Menge an Flüssigkeit, die bei jedem Stich in die Haut eingebracht wird, entsprechend hoch sein muss. Somit ist der Verbrauch von Bleichmittel verhältnismäßig hoch.

Aus EP 1 495 782 A1 ist eine Tätowierpistolenspitze für eine Tätowierpistole bekannt, die einen seitlich angebrachten Vorratsbehälter aufweist, der mit Tinte zum Tätowieren gefüllt ist. Der Vorratsbehälter ist in einem Bereich, in dem die Tätowierpistolenspitze beim Tätowieren mit der Hand gehalten wird, über eine Leitung mit dem Inneren der Tätowierpistolenspitze verbunden. Die Leitung mündet in ein Farbstoffreservoir, durch das die Nadel hindurch bewegt wird, um die Nadel mit ausreichend Tinte für die Tätowierung zu benetzen. Auch bei dieser Tätowierpistolenspitze ist der Verbrauch der in dem Vorratsbehälter angeordneten Flüssigkeit hoch.

Die Aufgabe der Erfindung ist es, eine Tätowierpistolenspitze zu schaffen, die zur Entfernung einer Tätowierung geeignet ist und einen verringerten Verbrauch eines für die Entfernung der Tätowierung benötigen Mittels aufweist.

Die Lösung der Aufgabe erfolgt erfindungsgemäß durch die Merkmale des Anspruchs 1.

Die erfindungsgemäße Tätowierpistolenspitze weist einen Spitzenkörper mit einem Nadelkanal zur Führung mindestens einer Nadel auf. Die Tätowierpistolenspitze weist ferner einen Vorratsbehälter zur Aufnahme einer Flüssigkeit auf. Erfindungsgemäß ist der Vorratsbehälter zumindest teilweise mit einem Hautirritationsmittel gefüllt. Zum Benetzen der Nadel mit dem Hautirritationsmittel ist der Vorratsbehälter über einen Zuführkanal mit dem Nadelkanal verbunden. Der Zuführkanal mündet hierfür in den Nadelkanal ein, so dass eine direkte Verbindung des Zuführkanals mit dem Nadelkanal besteht.

Da der Vorratsbehälter der erfindungsgemäßen Tätowierpistolenspitze Hautirritationsmittel enthält, ist die Tätowierpistolenspitze zur Entfernung von Tätowierungen oder Permanentmake-up geeignet. Erfindungsgemäß wird bei der Tätowierpistolenspitze ausgenutzt, dass es nicht erforderlich ist, größere Mengen Flüssigkeit, etwa 1 bis 1,5 mm tief in die Lederhaut einer menschlichen oder tierischen Haut einzubringen, wie dies bei Tätowierpistolenspitzen zum Tätowieren erforderlich ist. Es ist ausreichend, wenn im Vergleich zum üblichen Tätowieren verhältnismäßig wenig Hautirritationsmittel in die etwa 0,5 mm dicke Oberhaut (Epidermis) eingebracht wird, so dass die mit der Nadel mechanisch zerstörten Farbpigmentagglomerate in die Oberhaut eindringen können, ohne dass der natürliche Heilungsprozess der Haut dies verhindern würde. Aus der Oberhaut können die Farbpigmentagglomerate beim natürlichen Heilungsprozess der Haut mit abgetragen werden. Dadurch, dass das Hautirritationsmittel aus dem Vorratsbehälter über den Zuführkanal direkt in den Nadelkanal gelangt, sind Totvolumina, die bei herkömmlichen Tätowierpistolenspitzen zum Tätowieren für eine ausreichende Benetzung der Nadel erforderlich sind, vermieden. Insbesondere sind innerhalb des Spitzenkörpers vorgesehene Flüssigkeitsreservoirs nicht erforderlich. Stattdessen ist es ausreichend, wenn von dem Vorratsbehälter ein Tropfen in den Nadelkanal abgegeben wird, um die Nadel ausreichend mit dem Hautirritationsmittel zu benetzen. Die in dem Vorratsbehälter vorhandene Flüssigkeit kann somit nahezu vollständig in die Haut eingebracht werden, ohne dass relevante Restmenge in der Tätowierpistolenspitze verbleiben. Der Flüssigkeitsverbrauch an Hautirritationsmittel wird dadurch minimiert. Ferner kann der Spitzenkörper der Tätowierpistolenspitze im Vergleich zu gleich großen, herkömmlichen Tätowierpistolenspitzen stabiler ausgeführt sein, da auf Grund des reduzierten Hohlvolumens die Wandstärke des Spitzenkörpers größer sein kann.

Herbert P. Fiedler definiert in "Lexikon der Hilfsstoffe für Pharmazie, Kosmetik und angrenzende Gebiete", 4. Auflage, 1996 unter dem Stichwort "Hautirritationen", Hautreizstoffe als chemische Stoffe, die, wenn sie mit der Humanhaut in Berührung kommen, eine Hautreizwirkung ausüben. Hautreizstoffe im Sinne der vorliegenden Erfindung können somit eine primäre Reizwirkung aufweisen, die sofort nach dem Kontakt mit der Haut auftritt. Weniger geeignet im Sinne der vorliegenden Erfindung sind Hautreizstoffe, in denen sich Schädigungen erst nach länger dauernder Einwirkung des hautreizenden wirkenden Stoffes in unterschwelligen Dosen in Form eines degenerativen Ekzems bemerkbar machen. Besonders bevorzugt im Sinne der vorliegenden Erfindung werden somit Hautreizstoffe eingesetzt, die als Hautreizmittel (Hartirritationsmittel) dienen.

Fiedler loc. cit. definiert eine in den USA offiziell anerkannte Skala zur Prüfung der hautreizenden Wirkung. Die am stärksten hautreizenden Stoffe werden hier als korrosiv, hochgefährlich beschrieben, die mit einem Warnhinweis versehen sein müssen. Derartige Hautreizstoffe werden kaum für die vorliegende Erfindung eingesetzt werden, jedenfalls nicht in reiner Form. Gleiches gilt für primäre Hautreizstoffe, die ebenfalls hochgefährlich sind und Warnhinweise tragen müssen. Hautreizstoffe, die ein Potential für eine schwere Hautirritation haben, sollten ebenfalls im Sinne der vorliegenden Erfindung weniger geeignet sein. Hautreizstoffe, die kein hautreizendes Potential aufweisen, sind in Bezug auf die Wundheilung nicht von Bedeutung, es sei denn, dass die Anwesenheit als Füllstoff der Zelle die Wundheilung verzögert. Füllstoffe umfassen beispielsweise Feststoffe im Sinne der vorliegenden Erfindung, wie Kochsalz, Diamantstaub oder Quarzsand.

Die Hautreizstoffe können in fester Form und/ oder vorzugsweise in flüssiger Form auf die Hautoberfläche oder direkt in die Farbpigment-Agglomerat-Bruchstücke eingebracht werden. Besonders bevorzugt im Sinne der vorliegenden Erfindung ist es, entsprechende Hautreizstoffe einzusetzen, die aus verdünnten wässrigen Lösungen, Dispersionen und Emulsionen der Hautreizstoffe bestehen. Die chemische Natur der Hautreizstoffe ist nicht auf eine bestimmte Klasse von Verbindungen beschränkt. So können beispielsweise verdünnte wässrige Lösungen von Milchsäure, Natriumhydrazin, Kochsalz, Aminosäuren, Fruchtsäuren eingesetzt werden, die gegebenenfalls geringe Mengen an Oxidationsmitteln enthalten. Durch einfache Reihenversuche lässt sich das hautreizende Potential ohne weiteres feststellen und somit eine entsprechende Auswahl treffen.

Vorzugsweise weist der Vorratsbehälter eine Dosiereinrichtung auf, mit deren Hilfe das Hautirritationsmittel als Tropfen und/ oder kontinuierlich oder diskontinuierlich abgegeben werden kann. Dadurch ist es möglich, beispielsweise mit einer regelmäßigen Frequenz jeweils einen Tropfen von dem Vorratsbehälter in den Nadelkanal abzugeben. Ferner kann ein kontinuierlich fließender Flüssigkeitsstrom eingestellt werden. Auch ist es möglich, individuell eine bestimmte Menge des Hautirritationsmittels dem Nadelkanal zuzuführen. Die Abgabe des Hautirritationsmittels mit Hilfe der Dosiereinrichtung kann beispielsweise mechanisch über einen per Hand betätigten Kolben erfolgen. Alternativ kann beispielsweise Druckluft angeschlossen werden, die einen konstant einstellbaren Druck auf einen Kolben ausübt, so dass Einstellungen per Hand vermieden sind.

Die Dosiereinrichtung weist insbesondere einen Förderkolben auf, um das Hautirritationsmittel aus dem Vorratsbehälter in den Nadelkanal zu drücken. Ein nur durch Gewichtskräfte bestimmter Abgabestrom, der sich in Abhängigkeit von der im Vorratsbehälter noch verbliebenen Menge an Hautirritationsmittel ändert, ist somit nicht erforderlich. Vorzugsweise weist der Förderkolben eine Außenkontur auf, die in axialer Richtung sowohl an einer Innenkontur des Vorratsbehälters, sowie an eine Innenkontur des Zuführkanals angepasst ist. Der Förderkolben kann dadurch eine Formgestaltung aufweisen, dass der Förderkolben zumindest teilweise in den Zuführkanal hineinreicht. Dadurch kann der Vorratsbehälter sowie ggf. auch der Zuführkanal nahezu vollständig entleert werden, so dass nicht verwendete Restmengen des Hautirritationsmittels nahezu vollständig vermieden werden.

Der Zuführkanal und der Nadelkanal treffen sich vorzugsweise derart, dass der Zuführkanal und der Nadelkanal einen Winkel von 10° - 90°, insbesondere von 40° - 70°, bevorzugt von 50° - 60° einschließen. Der Zuführkanal ist hierbei insbesondere derart relativ zum Nadelkanal geneigt, dass der Zuführkanal zumindest teilweise in Richtung des zur Haut weisenden Endes des Spitzenkörpers weist. Durch diese Anordnung des Zuführkanals relativ zum Nadelkanal kann das Hautirritationsmittel zumindest teilweise mit Hilfe der Schwerkraft in den Nadelkanal gefördert werden. Ferner kann dadurch der Vorratsbehälter derart von dem Spitzenkörper abstehen, dass die Tätowierpistolenspitze leicht geführt werden kann, ohne dass der Vorratsbehälter die Sicht versperren würde oder das Greifen der Tätowierpistolenspitze erschwert würde.

Vorzugsweise ist der Vorratsbehälter mit dem Spitzenkörper zusätzlich über Stabilisierungsmittel, beispielsweise Streben und/ oder Stege verbunden. Der Vorratsbehälter kann dadurch eine entsprechend große Menge Hautirritationsmittel aufnehmen, ohne dass die Gefahr besteht, dass der Vorratsbehälter beispielsweise im Bereich des Zuführkanals von dem Spitzenkörper abbricht.

Um die Herstellung der Tätowierpistolenspitze zu vereinfachen, kann der Vorratsbehälter, der Zuführkanal und der Spitzenkörper sowie ggf. das Stabilisierungsmittel einstückig ausgeführt sind. Hierzu sind der Vorratsbehälter, der Zuführkanal, der Spitzenkörper sowie das Stabilisierungsmittel insbesondere durch Spritzguss hergestellt und bestehen vorzugsweise aus einem Kunststoff.

Vorzugsweise weist der Spitzenkörper ein mit der mindestens einen Nadel verbundenes Verbindungsmittel auf, mit dessen Hilfe die Nadel mit einem Bewegungselement der Tätowierpistole verbunden werden kann. Bei dem Bewegungselement handelt es sich beispielsweise um einen Stößel, eine Stange, einen Pleuel oder dgl., das eine im Wesentlichen translatorische Hin- und Her-, bzw. Auf- und Abbewegung bereitstellt, um die Nadel bzw. einen Satz mehrerer Nadeln in die Haut zu stechen und wieder herauszuziehen. Das Verbindungsmittel ist insbesondere als elastischer Körper ausgestaltet, der eine Einstecköffnung zum reibschlüssigen Verbinden mit dem Bewegungselement aufweist. Das Bewegungselement der Tätowierpistole kann somit einfach zur insbesondere starren Verbindung mit der Nadel in das Verbindungsmittel eingesteckt werden. Das Bewegungselement ist vorzugsweise starr, d. h. ohne Relativbewegung, mit der mindestens einen Nadel verbunden. Das Verbindungsmittel ist insbesondere innerhalb des Spitzenkörpers geführt, so dass ein Verkanten der Nadeln oder des Bewegungselementes beispielsweise innerhalb des Bewegungsmittels vermieden ist. Besonders bevorzugt ist das Verbindungselement derart ausgestaltet, dass das Bewegungselement in einem definierten und/ oder konstanten Abstand zu der Nadel mit der Nadel verbindbar ist. Beispielswiese sind hierfür die Nadel(n) sowie das Bewegungselement starr, d. h. ohne Relativbewegung, mit dem Verbindungsmittel verbunden.

Insbesondere weist der Spitzenkörper ein Befestigungsmittel zur Befestigung der Tätowierpistolenspitze an der Tätowierpistole auf. Das Befestigungsmittel kann hierbei beispielsweise als reibschlüssige Steckverbindung ausgeführt sein, indem beispielsweise ein Teil der Tätowierpistolenspitze reibschlüssig in einer Aufnahmeöffnung der Tätowierpistole eingesteckt ist. Vorzugsweise ist das Befestigungsmittel als Bajonnetverschluss ausgeführt, so dass eine besonders sichere und lösbare Verbindung gegeben ist. Auf Grund des Befestigungsmittels kann die Tätowierpistolenspitze als einzelnes Zubehörbauteil einer Tätowierpistole angeboten werden. Dies ermöglicht es, die Tätowierpistolenspitze als steriles bzw. steril verpacktes Bauteil, insbesondere als Wegwerfartikel anzubieten. Dadurch wird hohen Hygieneanforderungen genügt, wodurch die Gefahr beispielsweise von Infektion durch mit Keimen kontaminierten Nadeln reduziert wird.

Die Erfindung betrifft ferner eine Vorrichtung zum Entfernen einer Tätowierung, die ein Gehäuse aufweist, in dem eine Antriebseinrichtung angeordnet ist. Mit der Antriebseinrichtung ist ein Bewegungselement verbunden, das in dem Gehäuse geführt ist. Die Vorrichtung weist ferner eine Tätowierpistolenspitze auf, die insbesondere wie vorstehend ausgeführt, weitergebildet ist. Die Tätowierpistolenspitze ist mit dem Gehäuse und mit dem Bewegungselement verbunden. Hierbei wird die translatorische Bewegung insbesondere über eine geeignete Führung erzwungen. Der Spitzenkörper der Tätowierpistolenspitze und das Gehäuse der Vorrichtung zum Entfernen einer Tätowierung kann jedoch auch einstückig ausgestaltet sein und beispielsweise durch Kunststoffspritzguss hergestellt sein.

Die Antriebseinrichtung der Vorrichtung weist vorzugsweise einen Exzenter auf, der mit einem Abtriebselement der Antriebseinrichtung verbunden ist. Mit Hilfe des Exzenters kann eine rotatorische Bewegung des Abtriebselementes, bei der es sich beispielsweise um die Abtriebswelle eines Elektromotors handelt, in eine translatorische Bewegung des Bewegungselementes umgewandelt werden. Die Vorrichtung zum Entfernen einer Tätowierung umfasst also insbesondere eine mit einer Tätowierpistole verbundene Tätowierpistolenspitze, die ein Mittel zum Entfernen einer Tätowierung aufweist.

Besonders bevorzugt ist die Vorrichtung derart ausgestaltet, dass die Nadel der Tätowierpistolenspitze im ausgeschalteten Zustand der Antriebseinrichtung vollständig innerhalb des Spitzenkörpers angeordnet ist. Die Gefahr, sich an der Nadel der Tätowierpistolenspitze zu verletzen, wird hierdurch reduziert. Dies wird beispielsweise dadurch erreicht, dass mit Hilfe eines Rückstellelementes die Nadel und/ oder das Bewegungselement in einer Position gehalten ist, in der die Nadel(n) nicht aus dem Spitzenkörper heraus ragt, sondern sich innerhalb des Spitzenkörpers befinden. Als Rückstellelement ist beispielsweise eine Feder vorgesehen, die auf die mindestens eine Nadel und/ oder das Bewegungselement eine Kraft ausübt, welche von dem Ende der Tätowierpistolenspitze weg gerichtet ist. Das Rückstellelement kann auch eine Kraft auf den Exzenter ausüben, um ihn im ausgeschalteten Zustand der Antriebseinrichtung in einer Position zu halten, in welcher das Bewegungselement maximal weit entfernt von dem Ende der Tätowierpistolenspitze angeordnet ist.

Nachfolgend wird die Erfindung in einer bevorzugten Ausgestaltung unter Bezugnahme auf die anliegenden Zeichnungen näher erläutert.

Es zeigen:
- Fig. 1: eine schematische, maßstäbliche, teilweise transparente Seitenansicht einer Vorrichtung zum Entfernen einer Tätowierung,
- Fig. 2: eine schematische, maßstäbliche, perspektivische, teilweise transparente Seitenansicht einer Tätowierpistolenspitze,
- Fig.3: eine schematische, maßstäbliche, perspektivische, teilweise transparente Seitenansicht der Vorrichtung zum Entfernen einer Tätowierung mit der Tätowierpistolenspitze im zusammengebauten Zustand und
- Fig. 4: eine schematische maßstäbliche Draufsicht einer Antriebseinrichtung der Vorrichtung zum Entfernen einer Tätowierung.

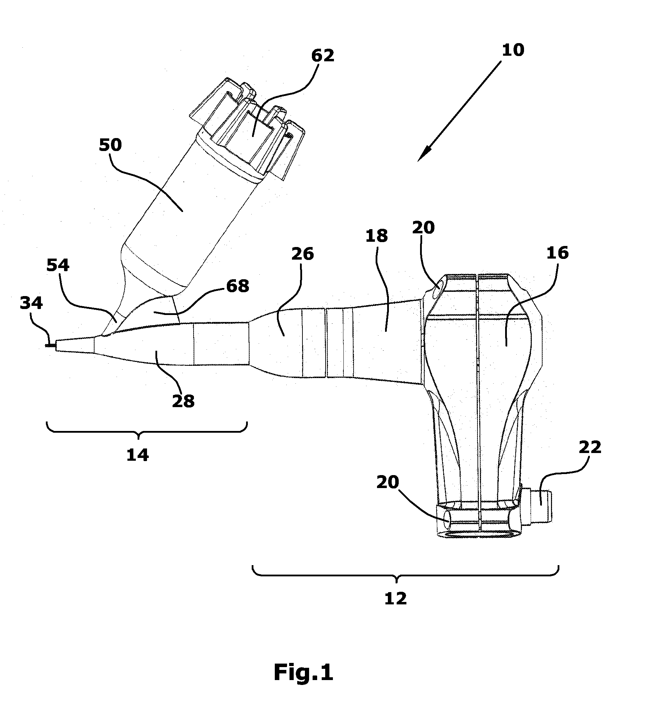

Die erfindungsgemäße Vorrichtung 10 zum Entfernen einer Tätowierung weist eine Tätowierpistole 12 und eine Tätowierpistolenspitze 14 auf, die mit der Tätowierpistole 12 verbunden ist (Fig. 1). Die Tätowierpistole 12 weist eine Oberschale 16 und eine Unterschale 18 auf, die ein Gehäuse der Tätowierpistole 12 ausbilden und über Schrauben 20 miteinander verbunden sind. In der Oberschale 16 ist ein Anschluss 22 angeordnet, über den eine Antriebseinrichtung 24 der Tätowierpistole 12 beispielsweise elektrisch oder pneumatisch angetrieben werden kann. Mit der Unterschale 18 ist eine Muffe 26 verbunden, über welche die Tätowierpistolenspitze 14 mit der Tätowierpistole 12 verbunden ist.

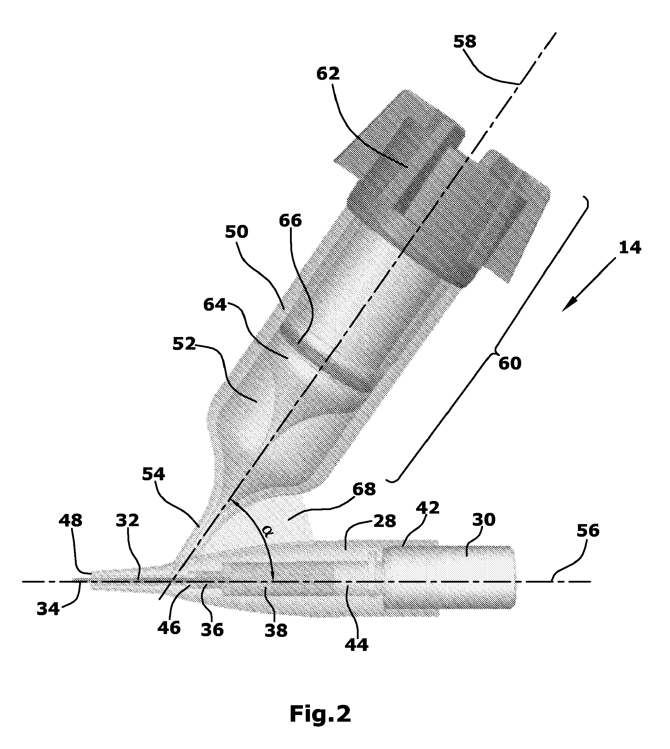

Die Tätowierpistolenspitze 14 weist einen Spitzenkörper 28 auf, der ein Befestigungsmittel 30 aufweist (Fig. 2). Im dargestellten Ausführungsbeispiel handelt es sich bei dem Befestigungsmittel 30 um einen Steckverbinder, der fest mit dem Spitzenkörper 28 verbunden ist und in die Muffe 26 der Tätowierpistole 12 reibschlüssig eingesteckt werden kann. Der Spitzenkörper 28 weist einen Nadelkanal 32 auf, in dem mehrere Nadeln 34 geführt sind. Bei den Nadeln 34 kann es sich um handelsübliche Tätowiernadeln handeln, die ggf. an ihrer Oberfläche aufgeraut sind. Die Nadeln 34 sind an ihrem von der Haut wegweisenden Ende beispielsweise durch Löten miteinander verbunden. Dieses Ende ist mit einem Verbindungselement 36 verbunden, das wiederum beispielsweise durch Vulkanisieren mit einem Verbindungsmittel 38 fest verbunden ist, wobei das Verbindungsmittel 38 wiederum mit einem Bewegungselement 40 der Tätowierpistole 12 verbunden werden kann.

Der Spitzenkörper 28 weist eine Befestigungsöffnung 42 auf, in welcher das Befestigungsmittel 30 beispielsweise über eine Presspassung fest eingesetzt ist. An die Befestigungsöffnung 42 schließt sich eine erste Führungsöffnung 44 an, in der das Verbindungsmittel 38 geführt ist. An die erste Führungsöffnung 44 schließt sich eine zweite Führungsöffnung 46 an, in der das Verbindungselement 36 geführt ist. Hieran schließt sich der Nadelkanal 32 an, der im Vergleich zu der ersten Führungsöffnung 44 und der zweiten Führungsöffnung 46 den geringsten Durchmesser aufweist. Der Nadelkanal 32 endet an einer Spitzenöffnung 48.

Die Tätowierpistolenspitze 14 weist ferner einen Vorratsbehälter 50 auf, der mit einem Hautirritationsmittel 52 gefüllt ist. Der Vorratsbehälter 50 geht ausgangsseitig in einen Zuführkanal 54 über, der in den Nadelkanal 32 einmündet. Die Einmündung des Zuführkanals 54 in den Nadelkanal 32 erfolgt unter einem Winkel a, der im dargestellten Ausführungsbeispiel a = 55° beträgt. D. h. eine Mittellinie 56 des Nadelkanals und eine Mittellinie 58 des Zuführkanals schließen einen Winkel von a = 55° ein.

Der Durchmesser des Nadelkanals 32 ist im dargestellten Ausführungsbeispiel derart gewählt, dass fünf handelsübliche Tätowiernadeln 34 gleichzeitig in dem Nadelkanal 32 geführt werden. Die Länge des Nadelkanals 32 ist im dargestellten Ausführungsbeispiel derart gewählt, dass die Nadeln 34 ca. 1,5 bis 2 mm aus der Spitzenöffnung 48 herausragen können. Der Durchmesser des Zuführkanals 54 ist vorzugsweise derart gewählt, dass der Strömungsquerschnitt des Zuführkanal 54 dem effektiven Strömungsquerschnitt des Nadelkanals 32 entspricht, so dass die Strömungsgeschwindigkeit in dem Zuführkanal 54 im Wesentlichen der Strömungsgeschwindigkeit des Hautirritationsmittels 52 in dem Nadelkanal 32 entspricht.

Der Vorratsbehälter 50 weist ferner eine Dosiereinrichtung 60 auf. Die Dosiereinrichtung 60 weist eine mit dem Vorratsbehälter 50 verschraubte Kappe 62 auf, die schnell und einfach mit der linken Hand betätigt werden kann, ohne dass die rechte Hand die Tätowierpistolenspitze 14 loslassen muss. Mit der Kappe 62 ist ein Kolben 64 verbunden, der eine Außenkontur aufweist, die einer Innenkontur des Vorratsbehälters 50 und einer Innenkontur des Zuführkanals 54 angepasst ist. Über einen Dichtring 66 des Kolbens 64 wird ein Austreten des Hautirritationsmittels 52 in Richtung der Kappe 62 vermieden.

Zur stabilen Verbindung des Vorratsbehälters 50 mit dem Spitzenkörper 28 ist ein Stabilisierungsmittel 68 vorgesehen, bei dem es sich im dargestellten Ausführungsbeispiel um einen Steg handelt, der sowohl mit dem Vorratsbehälter 50, mit dem Zuführkanal 54 als auch mit dem Spitzenkörper 28 verbunden ist. Bei dem Vorratsbehälter 50, dem Zuführkanal 54, dem Steg 68 und dem Spitzenkörper 28 handelt es sich um ein gemeinsames Bauteil, das durch Kunststoffspritzguss hergestellt ist.

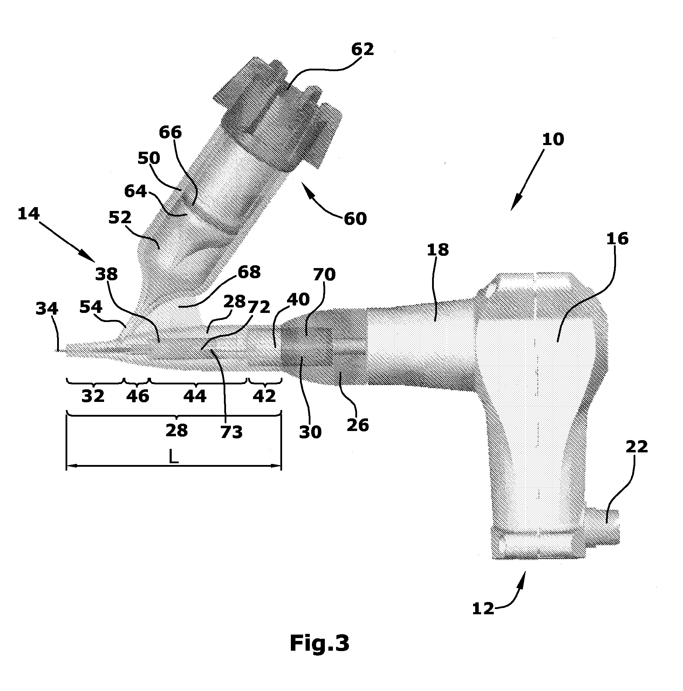

Im zusammengebauten Zustand (Fig. 3) ist die Tätowierpistolenspitze 14 über das Befestigungsmittel 30 mit der Tätowierpistole 12 verbunden. Zusätzlich oder alternativ kann zur Verbindung der Tätowierpistolenspitze 14 mit der Tätowierpistole 12 ein Bajonettverschluss vorgesehen sein. Im dargestellten Ausführungsbeispiel weist das Befestigungsmittel 30 eine Durchgangsöffnung 70 auf, durch die das Bewegungselement 40 hindurchgeführt ist. Das Bewegungselement 40 weist an seiner Spitze einen Verbindungsbereich 72 auf, der beispielsweise durch Riefen aufgeraut ist. Der Verbindungsbereich 72 ist in einer Einstecköffnung 73 des Verbindungsmittels 38 hineingesteckt und dadurch fest mit dem Verbindungsmittel 38 verbunden. Vorzugsweise weist der Spitzenkörper eine Länge L auf, die derart gewählt ist, dass bei der Verwendung der Vorrichtung 10 der Spitzenkörper 28 von der menschlichen Hand gegriffen wird. Dadurch kann die Vorrichtung 10 zum Entfernen einer Tätowierung vergleichbar zu einem Pinsel über die menschliche Haut geführt werden.

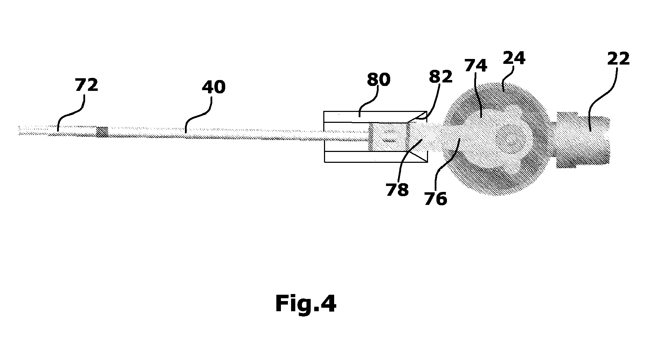

Die Antriebseinrichtung 24 der Tätowierpistole 12 weist einen Exzenter 74 auf, mit dessen Abtriebselement das Bewegungselement 40 über ein Pleuel 76 verbunden ist (Fig. 4). Der Pleuel 76 ist über ein zweites Verbindungsmittel 78 gelenkig mit dem Bewegungselement 40 verbunden. Das Bewegungselement 40, bzw. das zweite Verbindungsmittel 78 ist in einer Führung 80 geführt, so dass eine rein translatorische Bewegung erzwungen wird. Damit das Pleuel 76 nicht gegen die Führung 80 stößt, weist die Führung 80 eine zum Pleuel 76 weisende Anschrägung 82 auf. Die Führung 80 kann auch Bestandteil der Unterschale 18 des Gehäuses der Tätowierpistole 12 sein.

## Patentansprüche

1. Tätowierpistolenspitze für eine Tätowierpistole (12), mit
einem einen Nadelkanal (32) zur Führung mindestens einer Nadel (34) aufweisenden Spitzenkörper (28) und
einem mit dem Spitzenkörper (28) verbundenen Vorratsbehälter (50) zur Aufnahme einer Flüssigkeit,
**dadurch gekennzeichnet, dass**
der Vorratsbehälter (50) zumindest teilweise mit einem
Hautirritationsmittel (52) gefüllt ist und der Nadelkanal (32) mit dem Vorratsbehälter (50) über einen in den Nadelkanal (32) einmündenden Zuführkanal (54) zum Benetzen der Nadel (34) mit dem Hautirritationsmittel (52) verbunden ist.

2. Tätowierpistolenspitze nach Anspruch 1, **dadurch gekennzeichnet, dass** der Vorratsbehälter (50) eine Dosiereinrichtung (60) zur Abgabe eines Tropfens und/ oder zur kontinuierlichen oder diskontinuierlichen Abgabe des Hautirritationsmittels (52) aufweist.

3. Tätowierpistolenspitze nach Anspruch 2, **dadurch gekennzeichnet, dass** die Dosiereinrichtung (60) einen Förderkolben (64) aufweist, dessen Außenkontur in axialer Richtung an eine Innenkontur des Vorratsbehälters (50) sowie des Zuführkanals (54) angepasst ist.

4. Tätowierpistolenspitze nach einem der Ansprüche 1-3, **dadurch gekennzeichnet, dass** der Zuführkanal (54) und der Nadelkanal (32) einen Winkel (a) von 10° - 90°, insbesondere von 40° - 70° und bevorzugt von 50° - 60° einschließen.

5. Tätowierpistolenspitze nach einem der Ansprüche 1-4, **dadurch gekennzeichnet, dass** der Vorratsbehälter (50) und der Spitzenkörper (28) über ein Stabilisierungsmittel (68), insbesondere eine Strebe und/oder einen Steg, zusätzlich miteinander verbunden sind.

6. Tätowierpistolenspitze nach einem der Ansprüche 1 - 5, **dadurch gekennzeichnet, dass** der Vorratsbehälter (50), der Zuführkanal (54) und der Spitzenkörper (28) sowie ggf. das Stabilisierungsmittel (68) einstückig ausgebildet sind und insbesondere durch Spritzguss hergestellt sind.

7. Tätowierpistolenspitze nach einem der Ansprüche 1-6, **dadurch gekennzeichnet, dass** der Spitzenkörper (28) ein mit der mindestens einer Nadel verbundenes Verbindungsmittel (38) zur Verbindung mit einem Bewegungselement (40) der Tätowierpistole (12) aufweist, wobei das Verbindungsmittel (38) insbesondere als elastischer Körper mit einer Einstecköffnung (73) zum reibschlüssigen Verbinden mit dem Bewegungselement (40) ausgestaltet ist und der elastische Körper innerhalb des Spitzenkörpers (28) geführt ist.

8. Tätowierpistolenspitze nach Anspruch 7, **dadurch gekennzeichnet, dass** das Verbindungsmittel (38) derart ausgestaltet ist, dass das Bewegungselement (40) in einem definierten und/ oder konstanten Abstand zu der Nadel (34) mit der Nadel (34) verbunden ist.

9. Tätowierpistolenspitze nach einem der Ansprüche 1-8, **dadurch gekennzeichnet, dass** der Spitzenkörper (28) ein Befestigungsmittel (30) zur Befestigung mit einer Tätowierpistole (12) aufweist, das insbesondere als Bajonettverschluss ausgebildet ist.

10. Vorrichtung zum Entfernen einer Tätowierung, mit
einem Gehäuse (16, 18), in dem eine Antriebseinrichtung (24) angeordnet ist,
einem mit der Antriebseinrichtung (24) verbundenen und in dem Gehäuse (16, 18) geführten Bewegungselement (40) und
einer Tätowierpistolenspitze (14) nach einem der Ansprüche 1-8, die mit dem Gehäuse (16, 18) und mit dem Bewegungselement (40) verbunden ist.

11. Vorrichtung nach Anspruch 10, **dadurch gekennzeichnet, dass** die Antriebseinrichtung (24) einen mit einem Abriebselement verbundenen Exzenter (74) zum Umwandeln einer rotatorischen Bewegung des Abtriebselements in eine translatorische Bewegung des Bewegungselementes (40) mit Hilfe einer Führung (80) aufweist.

12. Vorrichtung nach Anspruch 10 oder 11, **dadurch gekennzeichnet, dass** im ausgeschalteten Zustand der Antriebseinrichtung (24) die Nadel (34) und/ oder das Bewegungselement (40) mit Hilfe eines Rückstellelementes in einer Position gehalten ist, in der die Nadel (34) vollständig innerhalb des Spitzenkörpers (28) angeordnet ist.

13. Vorrichtung nach einem der Ansprüche 10 - 12, **dadurch gekennzeichnet, dass** das Gehäuse (16, 18) und der Spitzenkörper (26) einstückig ausgeführt sind.
